# EUROPEAN PATENT APPLICATION

(11) **EP 2 077 103 A2**
(43) Date of publication of application: **08.07.2009**
(21) Application number: 09250021.4
(22) Date of filing: 06.01.2009
(51) Int. Cl.: A61K 6/08, A61K 6/083

(54) **Compositions for cementing prosthetic devices**

(30) Priority: 07.01.2008 US 969958
(71) Applicant: Kerr Corporation, Orange, CA 92867 (US)
(72) Inventor: Qian, Xuejun, Foothill Ranch, CA 92610 (US)
(74) Representative: Findlay, Alice Rosemary

(57) **Abstract**

A dental cementation kit for adhering a prosthetic device to tooth structure using a single-part light-curable self-adhering cement composition. The kit includes the light-curable single-part self-adhering cement composition, instruction for using same, and optionally an acidic etchant for etching tooth structure and/or an acidic etchant for etching a bonding surface of a dental prosthetic device. The light-curable single-part self-adhering cement composition comprises: (i) at least one acidic polymerizable monomer having at least one acidic moiety and at least one ethylenically unsaturated group, (ii) at least one non-acidic polymerizable monomer having at least one ethylenically unsaturated group, (iii) at least one photo-initiator, and (iv) at least one filler. The weight ratio of component (i) to component (ii) ranges from about 0.5:99.5 to about 70:30. The instructions for using the cement composition to attach a prosthetic device to tooth structure omit the steps of applying a primer and/or adhesive to the tooth structure and the bonding surface of the prosthetic device.

## Description

This invention relates to a cementation kit for cementing a dental prosthetic device to the tooth using a single-part light-curable self-adhering cement composition.

Prosthetic devices such as inlays, onlays, veneers, and crowns are used to replace carious or discolored tooth structure and restore the normal function of defective tooth structure. Although quite durable and easy to cement, prosthetic devices made of metallic material such as gold alloy, stainless steels, and base-metal alloys (e.g. nickel-chromium alloys) are optically opaque and non-aesthetic. Over the past two decades, tooth colored and aesthetic prosthetic devices made of ceramic materials, indirect composite resins, and metal oxide materials have increasingly become the choice of restorative materials for both dental patients and practitioners due to their unmatched aesthetics. Examples of ceramic materials include porcelain, feldspathic porcelain, aluminous porcelain, leucite reinforced ceramic material, lithium disilicate reinforced ceramic material, glass-infiltrated magnesia aluminate spinell, glass-infiltrated alumina, glass-infiltrated zirconia, and alumina. Examples of metal oxide materials include alumina, zirconia, and yttrium stabilized zirconia. The non-opaque and tooth colored restoration requires use of a resin cement to adhesively bond it to tooth structure because of resin cement's excellent aesthetic qualities (color matching ability and good translucency). However, current resin cements are hydrophobic and have no self adhesive properties, and therefore require a more complex cementation procedure including etching, priming/bonding, and cementing steps.

A typical cementation procedure for cementing a ceramic prosthetic device to tooth structure is as follows. If the patient wears a temporary prosthetic device, the temporary prosthetic device along with the temporary cement will be removed first. The tooth will be thoroughly cleaned typically using pumice cleaning and subsequently rinsed with water. The cementation process involves following steps: (1) the tooth is first etched with an acidic etchant to remove the smear layer on the tooth surface and also to create a more retentive surface for bonding; (2) a dental primer (for some adhesive systems) is applied to the tooth surface; (3) a dental adhesive is then applied to the tooth surface; (4) the dental adhesive is light-cured; (5) the ceramic bonding surface is air-abraded with aluminum oxide particles (optional); (6) the ceramic surface is then etched with hydrofluoric acid; (7) the ceramic surface is coated with a silane primer; (8) the ceramic surface is then coated with a primer/adhesive (for most cementation system); (9) the ceramic surface is bonded to the tooth with a light-curable or dual-curable (light-curable and self-curable) resin cement; and finally (10) the resin cement is hardened by light-curing or dual-curing (light-curing and self-curing). The current procedures for cementing a dental ceramic veneer restoration to tooth structure is rather cumbersome, involves many steps, and therefore is quite technique sensitive and time consuming.

It is highly desirable to simplify the above cementation procedures and shorten the chair time for the dental practitioner. This would also significantly reduce the chance for errors involved in the cementation procedure since less steps and components are involved.

The current invention provides a dental cementation kit comprising a light-curable single-part self-adhering cement composition, instructions for using the cement composition, and optionally one or two compositions selected from the group consisting of an acidic etchant for etching tooth structure, and an acidic etchant for etching a bonding surface of a dental prosthetic device;
wherein the light-curable single-component self-adhering cement composition comprises:
(i) at least one acidic polymerizable monomer having at least one acidic moiety and at least one ethylenically unsaturated group,
(ii) at least one non-acidic polymerizable monomer having at least one ethylenically unsaturated group,
(iii) at least one photo-initiator, and
(iv) at least one filler, wherein the weight ratio of component (i) to component (ii) ranges from about 0.5:99.5 to about 70:30; and
wherein the instructions for using the cement composition to attach a prosthetic device to tooth structure omit the steps of applying a primer and/or adhesive to the tooth structure and the bonding surface of the prosthetic device.

There is also disclosed a simplified method for adhering a prosthetic device to tooth structure using a single-part light-curable self-adhering cement composition. The method comprises the steps of: (1) adhering a bonding surface of the prosthetic device to tooth structure directly using a light-curable single-part self-adhering cement composition without first treating the tooth structure and the bonding surface of the prosthetic device with a primer/adhesive at the time of the adhering; and (2) hardening the cement composition by photo-curing the cement composition. The light-curable single-part self-adhering cement composition comprises: (i) at least one acidic polymerizable monomer having at least one acidic moiety and at least one ethylenically unsaturated group, (ii) at least one non-acidic polymerizable monomer having at least one ethylenically unsaturated group, (iii) at least one photo-initiator, and (iv) at least one filler. The weight ratio of component (i) to component (ii) ranges from about 0.5:99.5 to about 70:30.

The omission of the steps of applying a primer/adhesive to tooth structure and the bonding surface of the prosthetic device significantly simplifies the restorative procedures and will result in significant time savings for the dentists, especially when cementing multi-unit restorations, as is often the case for veneer cementation.

The method may further comprise the steps of etching the tooth structure with an acidic etchant composition and cleaning the etched tooth surface by rinsing with water prior to the adhering with the cement composition, with the acidic etchant composition comprising at least one acidic compound. Alternatively the adhering with the cement composition is without first etching the tooth structure with an acidic etchant.

In one embodiment the prosthetic device is selected from the group consisting of an inlay, an onlay, a veneer, a crown, an orthodontic bracket and an orthodontic band. The prosthetic device may be made of a non-opaque restorative material. The non-opaque restorative material may be selected from the group consisting of a cured thermoset composite resin, a thermoplastic material, a ceramic material and a metal oxide.

If the non-opaque restorative material is a ceramic material, the ceramic material may be selected from the group consisting of porcelain, feldspathic porcelain, aluminous porcelain, leucite reinforced ceramic material, lithium disilicate reinforced ceramic material, glass infiltrated magnesia aluminate spinell, glass-infiltrated alumina, and glass-infiltrated zirconia. Preferably the bonding surface of the ceramic prosthetic device is etched with an acidic etchant and subsequently cleaned prior to the adhering with the cement composition. The acidic etchant may comprise an acid selected from the group consisting of hydrofluoric acid and phosphoric acid. Alternatively the bonding surface of the ceramic prosthetic device is not etched with an acidic etchant prior to the adhering with the cement composition.

If the non-opaque restorative material is the metal oxide, the metal oxide may be selected from the group consisting of zirconia, yttrium stabilized zirconia, and alumina. Preferably the bonding surface of the metal oxide prosthetic device is not etched with an acidic etchant prior to the adhering with the cement composition.

If the prosthetic device is a veneer, the veneer is preferably made of a ceramic restorative material.

The prosthetic device may be produced by a CAD/CAM process.

In another embodiment the bonding surface of the prosthetic device is air-abraded with fine particles and subsequently cleaned prior to the adhering with the cement composition.

The invention will now be further described by way of the following detailed description.

A simplified method for adhering a prosthetic device to tooth structure using a single-part light-curable self-adhering cement composition comprises the steps of: (1) adhering the prosthetic device to tooth structure directly using a light-curable single-part self-adhering cement composition without first treating the tooth structure and the bonding surface of the prosthetic device with a primer/adhesive at the time of the adhering (i.e., at the time of cementation); and (2) hardening the cement composition by photo-curing the cement composition. In the method, the light-curable single-part self-adhering cement composition comprises: (i) at least one acidic polymerizable monomer having at least one acidic moiety and at least one ethylenically unsaturated group, (ii) at least one non-acidic polymerizable monomer having at least one ethylenically unsaturated group, (iii) at least one photo-initiator, and (iv) at least one filler. The weight ratio of component (i) to component (ii) ranges from about 0.5:99.5 to about 70:30.

For the acidic polymerizable monomer (i) of the dental cement composition, at least one acidic polymerizable monomer having at least one ethylenically unsaturated group and at least one acidic moiety can be used. Examples of ethylenically unsaturated groups include, but are not limited to, (meth)acrylate {(meth)acrylate = acrylate or methacrylate}, acrylamide, methacrylamide, and vinyl groups. The acidic moiety can be any acidic functional group. In one embodiment, the acidic moiety is selected from the group consisting of phosphorous-containing acidic moiety, carbon-containing acidic moiety, sulfur-containing acidic moiety, and boron-containing acidic moiety. Examples of acidic moieties include, but are not limited to, sulfonic acid, sulfinic acid, carboxylic acid, carboxylic acid anhydride, phosphonic acid or its derivative, and phosphoric acid or its derivative, with a derivative being a salt or ester of the respective acid.

In one embodiment, the acidic polymerizable monomer has at least one phosphorus-containing acidic moiety. Examples of phosphorus-containing acidic moieties include phosphonic acid or its derivative, and phosphoric acid or its derivative, such as the acidic moiety selected from the group consisting of 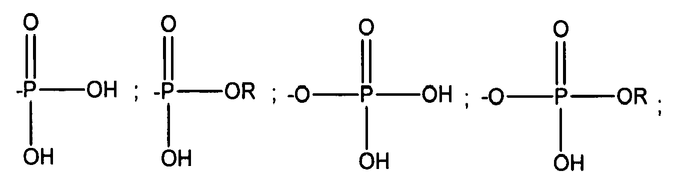 where R is an alkyl group, aryl group, or alkali metal ion.

Examples of acidic polymerizable monomers having at least one phosphorus-containing acidic moiety include, but are not limited to, phenyl methacryloxyethyl phosphate, glyceryldimethacrylate phosphate (GDMA-P), dipentaerithritol pentaacrylate phosphate, pentaerithritol triacrylate phosphate, methacryloyloxydecyl phosphate, hydroxyethylmethacrylate phosphate, and bis(hydroxyethylmethacrylate) phosphate, and combinations thereof. In one embodiment, the acidic polymerizable monomer is glyceryldimethacrylate phosphate (also called "glyceryldimethacrylate dihydrogen phosphate").

In another embodiment, the acidic monomer contains at least one carbon-containing acidic moiety. Examples of carbon-containing acidic moieties include, but are not limited to, carboxylic acid and carboxylic anhydride. Examples include, but are not limited to, maleic acid, itaconic acid, methacrylic acid, acrylic acid, polymerizable homopolymer or copolymer of an α, β - unsaturated carboxylic acid, maleic anhydride, 4-methacryloxyethyltrimellitic anhydride, 4-methacryloxyethyltrimellitic acid, and any addition product of mono- or di-anhydride compound with an hydroxyalkylmethacrylate compound. In one embodiment, the acidic polymerizable monomer is a polymerizable homopolymer or copolymer of an α, β - unsaturated carboxylic acid. Examples of polymerizable homopolymers or copolymers of an α, β - unsaturated carboxylic acid include, but are not limited to (meth)acrylated poly(acrylic acid), (meth)acrylated poly(acrylic acid) copolymer such as (meth)acrylated poly(acrylic acid-maleic acid) copolymer or (meth)acrylated poly(acrylic-maleic acid-itaconic acid) copolymer. In one embodiment, the acidic polymerizable monomer is selected from the group consisting of 4-methacryloxyethyltrimellitic anhydride and 4-methacryloxyethyltrimellitic acid. In another embodiment, the acidic polymerizable monomer is an addition product of mono- or di-anhydride compound with a hydroxyalkylmethacrylate compound. Examples of addition products of mono- or di-anhydride compound with a hydroxyalkylmethacrylate compound include, but are not limited to the addition product of pyromellitic acid anhydride and 2-hydroxyethyl methacrylate, the addition product of pyromellitic acid anhydride and glyceryl dimethacrylate, the addition product of 3,3',4,4'-benzophenonetetracarboxylic dianhydride and hydroxyethyl methacrylate, the addition product of phthalic anhydride and hydroxyethyl methacrylate, and the addition product of maleic anhydride and glyceryl dimethacrylate.

For the component (ii) of the cement composition, the non-acidic polymerizable monomer contains at least one ethylenically unsaturated group, but contains no acid moiety. Examples of ethylenically unsaturated groups include, but are not limited to, (meth)acrylate, acrylamide, methacrylamide, and vinyl groups. Examples of non-acidic polymerizable monomers include but not limited to the following: methyl (meth)acrylate, ethyl (meth)acrylate, propyl (meth)acrylate, butyl (meth)acrylate, hexyl (meth)acrylate, octyl (meth)acrylate, lauryl (meth)acrylate, decyl (meth)acrylate, tridecyl (meth)acrylate; 2-ethoxyethyl (meth)acrylate, 2'-ethoxy-2-ethoxyethyl (meth)acrylate, ethyleneglycol di(meth)acrylate, diethyleneglycol di(meth)acrylate, triethyleneglycol di(meth)acrylate (TEGDMA), tetraethyleneglycol di(meth)acrylate, polyethyleneglycol mono-(meth)acrylate, polyethyleneglycol di-(meth)acrylate, polypropyleneglycol mono-(meth)acrylate, polypropyleneglycol di-(meth)acrylate, polytetramethyleneglycol mono-(meth)acrylate, polytetramethyleneglycol di-(meth)acrylate, hexanediol di(meth)acrylate, trimethyloylpropane tri(meth)acrylate, ethoxylated trimethyloylpropane tri(meth)acrylate (ETMPTA), UDMA (reaction product of 2-hydroxyethyl methacrylate with 2,4,4 - trimethylhexane diisocyanate), ethoxylated bisphenol A dimethacrylate ("EBPADMA-n", n = total number of moles of ethylene oxide in the molecule, with 2-20 units being preferred), tetrahydrofurfuryl (meth)acrylate, 2,2-bis[4-(2-hydroxy-3-methacryloylpropoxy)-phenyl]-propane (Bis-GMA), hydroxyethyl (meth)acrylate (HEMA), hydroxypropyl (meth)acrylate, hydroxybutyl (meth)acrylate; glyceryl di(meth)acrylate (GDMA), glyceryl mono(meth)acrylate, N,N'-methylenebis(acrylamide), N,N'-ethylenebis(acrylamide), and N,N'-butylenebis(acrylamide), and combinations thereof. In one embodiment, component (ii) comprises at least one polymerizable monomer having at least one hydroxyl group. Examples of hydroxyl-containing polymerizable monomers include, but are not limited to, hydroxyethyl (meth)acrylate (HEMA), hydroxypropyl (meth)acrylate, hydroxybutyl (meth)acrylate, glyceryl di(meth)acrylate (GDMA), glyceryl mono(meth)acrylate, and 2,2-bis[4-(2-hydroxy-3-methacryloylpropoxy)-phenyl]-propane (Bis-GMA).

The weight ratio of component (i) to component (ii) ranges from about 0.5:99.5 to about 70:30. In one embodiment, the weight ratio of component (i) to component (ii) ranges from about 5:95 to about 60:40. In one embodiment, the weight ratio of component (i) to component (ii) ranges from about 10:90 to about 50:50. In one embodiment, the weight ratio of component (i) to component (ii) ranges from about 15:85 to about 50:50.

The photoinitiator (iii) can be any compound that would generate free radicals upon exposure to a light source and cause the polymerization or hardening of the composition. The light source can be any dental curing light that emits light in the visible or ultraviolet range. Examples of photoinitiators include, but are not limited to, benzoin, benzoin ethers and esters, 2,2-diethoxy acetophenone, diketone compounds such as camphorquinone and 1-phenyl-1,2-propanedione, monoacylphosphine oxide, bisacylphosphine oxide, diaryliodonium salt, and triarylsulfonium salt, and combinations thereof. Additionally, a coinitiator can be used together with a photoinitiator to enhance curing efficiency. Coinitiators include tertiary amine and sulfinate compounds. Examples of coinitiators include, but are not limited to, ethyl 4-(N,N-dimethylamino) benzoate, 4-(N,N-dimethylamino) benzoic acid, 4-(N,N-dimethylamino) benzonitrile, 4-(N,N-dimethylamino) benzaldehyde, 2-(ethylhexyl)-4-(N,N-dimethylamino) benzoate, N,N-dimethylaminoethyl methacrylate, N,N-dimethylaminophenethyl alcohol, sodium benzenesulfinate, and sodium toluenesulfinate. In one embodiment, a photoinitiator system includes the combination of camphorquinone and a tertiary amine. Examples of tertiary amines include, but are not limited to, ethyl 4-(N,N-dimethylamino) benzoate, 4-(N,N-dimethylamino) benzoic acid, 4-(N,N-dimethylamino) benzonitrile, 4-(N,N-dimethylamino) benzaldehyde, 2-(ethylhexyl)-4-(N,N-dimethylamino) benzoate, N,N-dimethylaminoethyl methacrylate, N,N-dimethylaminophenethyl alcohol. In another embodiment, a photoinitiator system includes the combination of camphorquinone and bisacylphosphine oxide or monoacylphosphine oxide. In one embodiment, a photoinitiator may be present at a concentration of about 0.01 % (w/w) to about 10% (w/w) of the composition. In another embodiment, a photoinitiator may be present at a concentration of about 0.05% (w/w) to about 5% (w/w) of the composition.

For component (iv), one or more fillers can be incorporated into the cement composition. Examples of fillers include, but are not limited to, inorganic metal, salt, oxide, fluoride, nitride, silicate glass, aluminosilicate glass, aluminoborosilicate glass, fluoroaluminosilicate glass, quartz, fumed silica, colloidal silica, precipitated silica, zirconia-silica, polymeric filler, and/or polymerized composite fillers with inorganic particles. In one embodiment, inorganic fillers for increased x-ray contrast ability include metals, salts, oxides, fluorides, silicate glass, aluminosilicate glass, aluminoborosilicate glass, and fluoroaluminosilicate glass containing elements of high atomic number such as Sr, Y, Zr, Ba, La, Hf, Zn, Bi, W, and rare earth metals, and combinations of these. Examples include but are not limited to barium sulfate, silver, strontium fluoride, barium fluoride, ytterbium fluoride, yttrium fluoride, barium tungstate, zinc oxide, bismuth(III) oxide, bariumaluminosilicate, bariumaluminoborosilicate, strontiumaluminosilicate, bariumfluoroaluminosilicate, strontiumfluoroaluminosilicate, strontiumzincfluoroaluminosilicate, and zincaluminosilicate. Fumed silica, colloidal silica, or precipitated silica can also be incorporated to improve the dispersion of the filler, as well as the rheological and handling properties of the composition. Examples of fumed, colloidal silicas are Aerosil^{®} series such as OX-50, OX-130, and OX-200 silica sold by Degussa (Ridgefield Park, NJ), and Cab-O-Sil^{®} M5 and Cab-O-Sil^{®} TS-530 silica sold by Cabot Corp (Tuscola, IL). The filler may also include nanoparticles such as those obtained through a solgel process. Examples include those disclosed in U.S. Patent Nos. 4,567,030 and 5,609,675. Mixtures of different fillers can be used. For inorganic fillers, the surface of the filler may be treated or coated with a coupling agent, such as gamma-methacryloyloxypropyltrimethoxysilane (MPTMS), that enhances the interfacial bonding between the filler and resin matrix and improves mechanical properties. In one embodiment, the mean particle size of the filler is less than about 50 microns. In another embodiment, the mean particle size of the filler is less than about 20 microns. In another embodiment, the mean particle size of the filler is less than about 10 microns. The concentration of total filler(s) ranges from about 15% (w/w) to about 90% (w/w) of the cement composition. In one embodiment, the concentration of total filler(s) ranges from about 30% (w/w) to about 85% (w/w) of the cement composition. In one embodiment, the concentration of total filler(s) ranges from about 50% (w/w) to about 80% (w/w) of the cement composition. In one embodiment, the concentration of component (iv) in the cement composition is in an amount so that the consistency of 0.5gm of the composition will be in the range of about 10 mm - about 50 mm. In one embodiment, the concentration of component (iv) in the cement composition is in an amount so that the consistency of 0.5gm of the composition will be in the range of about 10 mm - about 45 mm. In another embodiment, the concentration of component (iv) in the cement composition is in an amount so that the consistency of 0.5gm of the composition will be in the range of about 15 mm - about 40 mm. The consistency test is conducted at room temperature (23.5 ± 1 °C) per the following procedures: place 0.50±0.01 grams of the cement composition on a 75 x 50 mm (1mm thick) glass slide (Corning Inc., MA); gently place a second 75 x 50 mm (1mm thick) glass slide and a 108±1 gram weight (total weight including top glass slide: 117 ± 1.0 grams) over the cement composition, assuring even weight distribution; and after 10 minutes, measure the major and minor diameters of the slumped cement mass in millimeters (mm). The average of the major and minor diameters will be the consistency of the cement composition.

The cement composition may further comprise one or more ingredients selected from the group consisting of a solvent, colorant, stabilizer, UV absorber, a fluoride-releasing compound, and antimicrobial additive. For the solvent, any solvent can be used. In one embodiment, a solvent is selected from the group consisting of ethanol, water, methanol, acetone, methyl ethyl ketone, isopropanol, and t-butanol, and any combination thereof. In another embodiment, the solvent is selected from the group consisting of ethanol, water, isopropanol, and t-butanol, and any combination thereof. In one embodiment, the concentration of solvent is in the range of about 0% - 20% by weight. In another embodiment, the concentration of solvent is in the range of about 0% - 10% by weight. In another embodiment, the composition comprises no solvent. The colorant is used to achieve desired shade and can be an inorganic pigment or an organic dye. The stabilizer is a polymerization inhibitor or retarder to improve the shelf stability of the adhesive composition. The most commonly used stabilizers include 2,6-di-(*tert*-butyl)-4-methylphenol ("BHT") and 4-methoxyphenol ("MEHQ"). The UV absorber is used to improve the color stability of the adhesive composition upon exposure to UV light. An example of UV absorber is 2-hydroxy-4-methoxybenzophenone ("UV-9"). A fluoride- releasing compound is any fluoride-containing substance that can release fluoride into saliva, water, or surrounding dentition. Examples of fluoride-releasing compounds include, but are not limited to, sodium fluoride, strontium fluoride, sodium hexafluorosilicate, zinc hexafluorosilicate, rare earth metal fluoride such as ytterbium fluoride, a salt formed by an amine and hydrofluoric acid, a complex formed by an amine and BF₃, and a combination thereof. Examples of antimicrobial additives include, but are not limited to, benzalkonium chloride, iodoform, eugenol, zinc oxide, triclosan, alkyl 4-hydroxybenzoate, silicate glass powder containing silver and/or zinc, and zeolite powder containing silver and/or zinc ion(s). Useful antibacterial zeolites and their preparation are disclosed in U.S. Patent Nos. 4,911,899 and 4,775,585.

The omission of the steps of applying a primer/adhesive for tooth structure and the bonding surface of a prosthetic device simplifies the restorative procedures and will result in significant time savings for the dentists, especially when cementing multi-unit restorations, as is often the case for veneer cementation.

In one embodiment of the method, the tooth surface is etched with an acidic etchant and cleaned prior to cement application. The etched tooth surface is usually cleaned by rinsing with water prior to cement application. The acidic etchant comprises at least one acidic compound. Examples of suitable acidic compounds include, but are not limited to, phosphoric acid, phosphonic acid, maleic acid, nitric acid, or citric acid. In one embodiment, the acidic etchant used to etch tooth comprises phosphoric acid. An example of acidic etchant is Kerr Gel Etchant (Kerr, CA) containing phosphoric acid.

In one embodiment of the method, the step of etching the tooth surface prior to cement application is further omitted. Therefore both the steps of etching the tooth surface and applying a primer/adhesive to tooth surface are omitted prior to cement application.

The current light-curable one-part cement composition can be used to cement a variety of prosthetic devices or orthodontic appliances. In one embodiment, the prosthetic device is selected from the group consisting of an inlay, onlay, a veneer, and a crown. In one embodiment, the prosthetic device is selected from the group consisting of an orthodontic bracket and an orthodontic band. In one embodiment, the material used to make the prosthetic device is non-opaque so that light can penetrate through the prosthetic device and initiate the polymerization of the light-curable self-adhering cement composition. Here, "non-opaque" means the prosthetic material does not completely block the light. In one embodiment, the non-opaque prosthetic material of 1mm thickness will have an optical opacity of less than about 99%. The opacity can be measured with a spectrophotometer (model SP62 or SP64 or SP66, X-RITE Inc., MI). In one embodiment, the non-opaque prosthetic material of 1mm thickness will have an optical opacity of less than about 95%. In one embodiment, the non-opaque prosthetic material of 1mm thickness will have an optical opacity of less than about 90%. Examples of non-opaque materials used to make the prosthetic device include, but are not limited to, a cured thermoset composite resin, a thermoplastic material, ceramic material, and a metal oxide.

In one embodiment, the prosthetic device is made of a ceramic restorative material. Examples of ceramic restorative materials include porcelains, feldspathic porcelains, aluminous porcelains, lithium disilicate reinforced ceramic material such as IPS Eris (Vivadent, NY), leucite reinforced ceramic materials such as IPS Empress and ProCAD® (Vivadent, NY), glass-infiltrated magnesia aluminate spinell, glass-infiltrated alumina, glass-infiltrated zirconia. When the prosthetic device is made of a ceramic restorative material, the bonding surface of the prosthetic device is optionally processed by at least one step selected from the group consisting of sandblasting the surface of the prosthetic device and cleaning the surface; etching the surface of the prosthetic device with an acidic etchant and cleaning the surface; and a combination thereof. Any of above surface preparation can be performed either in a dental lab (by a dental technician) or in a dental office (by a dentist or dental assistant). In one embodiment, the step of sandblasting the ceramic bonding surface is omitted. In one embodiment, the internal surface (or bonding surface) of prosthetic device is sandblasted and cleaned prior to cement application. In another embodiment, the method comprises the step of etching the bonding surface of the prosthetic device and subsequent cleaning of the surface prior to cement application. The etching can be carried out in a dental lab and/or in the dental office. If the etching is carried out in the dental lab, then re-etching the prosthetic device is no longer necessary in the dental office unless a new bonding surface is created in the dental office. A new bonding surface can be created by abrading the bonding surface with a dental bur or sandblasting the bonding surface with fine inorganic particles such as aluminum oxide particles with an average particle size of about 10-250 microns. The acidic etchant used to etch the ceramic surface comprises an acid. In one embodiment, the acidic etchant used to etch the ceramic surface comprises an acid selected from the group consisting of hydrofluoric acid and phosphoric acid. In one embodiment, the acidic etchant used to etch the ceramic surface comprises hydrofluoric acid. With the method, the step of treating the ceramic surface with a primer/adhesive is omitted from the cementation procedures at the time of cementation. When the dentist receives the ceramic prosthetic device from the dental lab, it is possible that a primer (e.g. a silane primer) may have already been coated on the bonding surface of the ceramic prosthetic device by the dental lab. However, at the time of cementation, during the try-in step to try both the fit and color of the prosthetic device using a trying gel, the bonding surface will get contaminated and requires cleaning prior to cementation. Current cementation instructions will instruct the dentists to clean the bonding surface by applying phosphoric acid etchant and/or rinsing with water, and to reapply a ceramic primer (or a silane primer) or the combination of a ceramic primer and an adhesive. Or alternatively, current cementation instructions will instruct the dentist to sandblast the bonding surface to create a new surface, etch the new bonding surface with an acidic etchant (e.g. hydrofluoric acid etchant), and treat the surface with a ceramic primer or the combination of a primer and an adhesive. Both protocols comprise the steps of applying a primer or the combination of a primer and an adhesive. In the method using the single-part light-curable self-adhering cement composition, a primer/adhesive (a primer and/or an adhesive) is no longer needed at the time of cementation and the step of applying a primer/adhesive to the bonding surface of the prosthetic device is omitted from the cementation procedure at the time of cementation. In one embodiment, the cementation procedure is further simplified in that both the steps of etching the bonding surface of the prosthetic device and applying a primer/adhesive to the bonding surface of the prosthetic device are omitted at the time of cementation.

In one embodiment, the prosthetic device is made of a metal oxide. Examples of metal oxide restorative materials include, but are not limited to zirconia, yttrium stabilized zirconia, and alumina. Examples of zirconia-based restorative materials include Lava™ (3M ESPE, MN), Cercon® (Dentsply, DE), and Porcera® Zirconia (Nobel Biocare USA, CA). Examples of alumina-based restorative materials include, but are not limited to, Vita® in-Ceram® alumina (Vident, CA) and Porcera® alumina (Nobel Biocare USA, CA). In one embodiment of the inventive method, the prosthetic device is made of a metal oxide restorative material and the bonding surface is processed by sandblasting the surface and subsequent cleaning prior to cement application with both steps of etching and applying a primer/adhesive omitted. The sandblasting is used to enhance the adhesion by increasing the surface area by abrading the surface with fine inorganic particles such as aluminum oxide particles with an average particle size of about 10-250 microns, more preferably about 25-100 microns. The cleaning after sandblasting can be achieved by rinsing with water.

In one embodiment, the prosthetic device is made of a cured thermoset composite resin. Examples of thermoset composite resins include, but are not limited to, belleGlass® NG (Kerr, CA) and Sinfony™(3M ESPE, MN). In one embodiment, the composite resin comprises one or more fillers or fibers. The curing of composite resin is achieved by heat-curing and/or light-curing. When the prosthetic device is made of a cured thermoset composite resin restorative material, the bonding surface of the prosthetic device is optionally processed by at least one step selected from the group consisting of sandblasting the surface of the prosthetic device and cleaning the surface; etching the surface of the prosthetic device with an acidic etchant and cleaning the surface; and a combination thereof. Any of above surface preparations can be performed either in a dental lab (by a dental technician) or in a dental office (by a dentist or dental assistant).

In another embodiment, the prosthetic device is made of a thermoplastic material. Examples of thermoplastic materials include, but are not limited to, polycarbonate, PEEK {poly(ether-ether-ketone)}, and ABS (acrylonitrile-butadiene-styrene copolymer). The thermoplastic material can comprise one polymer or a blend of two or more polymers. The thermoplastic material can also be a thermoplastic material filled with one or more fillers or fibers. When the prosthetic device is made of a thermoplastic restorative material, the bonding surface of the prosthetic device is optionally processed by at least one step selected from the group consisting of sandblasting the surface of the prosthetic device and cleaning the surface; etching the surface of the prosthetic device with an acidic etchant and cleaning the surface; and a combination thereof. Any of above surface preparations can be performed either in a dental lab (by a dental technician) or in a dental office (by a dentist or dental assistant).

In one embodiment, the prosthetic device is produced by a CAD (computer-aided design)/CAM (computer-aided machining) process and the device is milled by a CAM machine. Examples of restorative material used for CAD/CAM processing include Vitablocs® Mark II (Vident, CA), ProCAD® (Vivadent, NY), and Paradigm™ MZ100 (3M ESPE, MN).

In a preferred embodiment, the prosthetic device is a ceramic veneer restoration and the a simplified cementation method of using a light-curable self-adhering cement composition to attach dental ceramic veneer(s) to tooth structure is achieved by omitting the steps of applying a primer/adhesive to the tooth structure and the bonding surface of the veneer at the time of cementation. In another embodiment, the veneer cementation procedure is further simplified by omitting both steps of etching the tooth surface and applying a primer/adhesive to the tooth surface at the time of cementation. In another embodiment, the veneer cementation procedure is further simplified by omitting both steps of etching the bonding surface of the veneer(s) and applying a primer/adhesive to the bonding surface of the veneer(s) at the time of cementation.

A dental cement kit comprises the light-curable single-part self-adhering cement composition, instructions for using the cement composition, and optionally one or two compositions selected from the group consisting of an acidic etchant for etching dentin/enamel, and an acidic etchant for etching the bonding surface of a dental prosthetic device. The instructions for using the cement composition to attach the prosthetic device to tooth structure omit the steps of applying a primer/adhesive (primer/adhesive = primer and/or adhesive) to tooth surface and the bonding surface of the prosthetic device. The cementation kit does not include a dental primer or adhesive.

All the discussion in previous paragraphs concerning the single-part self-adhering cement composition in relation to the method apply to the light-curable single-part self-adhering cement composition in the cement kit.

The acidic etchant comprises at least one acidic compound. In one embodiment, the acidic etchant used to etch the tooth surface comprises an acid selected from the group consisting of phosphoric acid, citric acid, maleic acid, phosphonic acid, and nitric acid. In one embodiment, the acidic etchant used to etch the tooth surface comprises phosphoric acid. In one embodiment, the acidic etchant used to etch bonding surface of the prosthetic device comprises an acid selected from the group consisting of hydrofluoric acid, phosphoric acid, citric acid, maleic acid, phosphonic acid, and nitric acid. In another embodiment, the acidic etchant used to etch bonding surface of the prosthetic device comprises hydrofluoric acid.

In one embodiment, the acidic etchant for etching dentin/enamel and/or the acidic etchant for etching the bonding surface of a prosthetic device is omitted from the kit. In one embodiment, both the acidic etchant for etching dentin/enamel and the acidic etchant for etching the bonding surface of a prosthetic device are omitted from the kit. In one embodiment, instructions for using the cement composition further omits at least one of the steps selected from the group consisting of the step of etching dentin/enamel and the step of etching the bonding surface of a prosthetic device. In one embodiment, the instructions for using the cement composition omits both the step of etching dentin/enamel and the step of etching the bonding surface of a prosthetic device.

In one embodiment, the cement kit is used to attach a prosthetic device to tooth surface wherein the prosthetic device is selected from the group consisting of an inlay, an onlay, a veneer, and a crown. In one embodiment, the prosthetic device is made of a material selected from the group consisting of a cured thermoset composite resin, a thermoplastic material, ceramic material, and a metal oxide. In one embodiment, the cement kit is a dental veneer cementation kit that is used to attach veneer(s) to tooth surface. In on embodiment, the veneer is made of a ceramic restorative material. In another embodiment, the cement kit is used to attach a prosthetic device to tooth surface wherein the prosthetic device is selected from the group consisting of an orthodontic bracket and an orthodontic band.

According to an embodiment, the bond strength of the single-part self-adhering cement composition to both the tooth structure and the prosthetic device is at least about 10 MPa. According to another embodiment, the adhesive strength of the self-adhering composition to both the tooth structure and the prosthetic device is at least about 15 MPa.

The following examples are illustrative of the invention.

### EXAMPLES

Abbreviations for materials used in all examples:
AHPMA: 3-acryloyloxy-2-hydroxy-propylmethacrylate
Bis-GMA: 2,2-bis[4-(2-hydroxy-3-methacryloylpropoxy)-phenyl]-propane
CQ: camphorquinone
EDMAB: ethyl 4-(N,N-dimethylamino) benzoate
ETMPTA: ethoxylated trimethylolpropane triacrylate with 3 moles of ethylene oxide
GDMA: glyceryldimethacrylate
GDMA-P: glyceryldimethacrylate phosphate or glyceryldimethacrylate dihydrogen phosphate
HEMA: hydroxyethyl methacrylate
MEHQ: 4-methoxyphenol
ODMAB: 2-(ethylhexyl)-4-(N,N-dimethylamino) benzoate
PMGDMA: the addition product of pyromellitic acid anhydride and glyceryl dimethacrylate.
TEGDMA: triethyleneglycol dimethacrylate
ST-OX-50: fumed silica OX-50 surface treated with γ-methacryloyloxypropyltrimethoxysilane
ST-BAS: bariumaluminoborosilicate filler that has a mean particle size of 2 micron and its surface was treated with γ-methacryloyloxypropyltrimethoxysilane
TS-530: surface treated fumed silica or colloidal silica sold by Cabot Corp.
UDMA: reaction product of 2-hydroxyethyl methacrylate with 2,4,4 -trimethylhexane diisocyanate
UV-9: 2-hydroxy-4-methoxybenzophenone.

### EXAMPLE 1

First, a homogeneous resin mixture (**1A**) was made of the following ingredients: 19.73% w/w of GDMA-P, 24.66% w/w of HEMA, 16.77% w/w of GDMA, 4.93% w/w of ETMPTA, 32.56% w/w of Bis-GMA, 0.99% w/w of ODMAB, 0.30% w/w of CQ, and 0.06% w/w of MEHQ. Then, a cement paste composition was made by blending the following ingredients together: 32.00% w/w of above resin mixture (**1A**), 1.74% w/w of ST-OX-50, 2.00% w/w of TS-530, and 64.26% w/w of ST-BAS. The cement composition had a consistency of 35mm.

The enamel bond strength test was conducted as follows: Bovine enamel specimens were embedded in cold-cure acrylics. A set of 6 specimens were prepared for each group. The enamel surface was then prepared with a fine diamond bur to create a new surface, and the surface was cleaned by rinsing with water and dried with compressed air from a dental air syringe for about 3 seconds. Without etching the enamel surface with an etchant or conditioning the enamel surface with a primer/adhesive, the bovine enamel surface was then held securely by a bonding jig (Ultradent Inc., UT) with a cylindrical mold (Φ = 2.38 mm). The cement composition was condensed inside the mold, and light-cured for 30 seconds using an Optilux™ 501 (Kerr, CA) dental curing light. After conditioning in 37°C water for 20-24 hours, the bond strength was tested on an Instron mechanical tester (Model 4467, Instron, MA) in shear mode using a notched (semi-circular) edge at a crosshead speed of 1.0 mm/min. An enamel bond strength of 16.7 ± 2.8 MPa was obtained.

Dentin bond strength test was also conducted according to the following method: Extracted human teeth were embedded in cold-cure acrylics. A set of six specimens were prepared for each group. A low speed diamond saw was used to remove the crown and expose the occlusal dentin. The dentin substrates were polished with 240-grit and subsequently 600-grit SiC paper, rinsed thoroughly with water, and air dried briefly. Without etching the dentin surface with an etchant or conditioning the dentin surface with a primer/adhesive, a plastic mold with an inner diameter of 2.38mm was securely placed over the dentin surface. The cement composition was condensed inside the mold, and light-cured for 30 seconds using an Optilux™ 501 (Kerr, CA) dental curing light. After conditioning in 37°C water for 20-24 hours, the bond strength was tested on an Instron mechanical tester (Model 4467, Instron, MA) using shear force. A dentin bond strength of 18.8 ± 4.7 MPa was obtained.

In this example, the enamel surface or the dentin surface was not etched with an etchant or pre-conditioned with an adhesive before bonding with the cement composition. The self-adhesiveness of the cement composition toward both dentin and enamel was clearly demonstrated.

### EXAMPLE 2

First, a homogeneous resin mixture (**2A**) was made of the following ingredients: 24.66% w/w of GDMA-P, 29.60% w/w of HEMA, 14.80% w/w of GDMA, 4.93% w/w of ETMPTA, 24.66% w/w of Bis-GMA, 0.99% w/w of ODMAB, 0.30% w/w of CQ, and 0.06% w/w of MEHQ. Then, a cement paste composition was made by blending the following ingredients together: 32% w/w of above resin mixture (**2A**), 1.74% w/w of ST-OX-50, 2.00% w/w of TS-530, and 64.26% w/w of ST-BAS. The cement composition had a consistency of 37mm. The enamel and dentin bond strength tests were conducted using this cement composition by following the procedures in Example 1. An enamel bond strength of 19.1 ± 2.2 MPa and a dentin bond strength of 23.4 ± 6.4 MPa were obtained. The aged cement composition after being subjected to accelerated aging at 37°C for 14 weeks yielded an enamel bond strength of 20.9 ± 2.1 MPa and a dentin bond strength of 17.0 ± 5.5 MPa.

### EXAMPLE 3

First, a homogeneous resin mixture (**3A**) was made of the following ingredients: 29.60% w/w of GDMA-P, 29.60% w/w of HEMA, 9.87% w/w of GDMA, 4.93% w/w of ETMPTA, 24.66% w/w of Bis-GMA, 0.99% w/w of ODMAB, 0.30% w/w of CQ, and 0.06% w/w of MEHQ. Then, a cement paste composition was made by blending the following ingredients together: 27% w/w of above resin mixture (**3A**), 1.87% w/w of ST-OX-50, 3.00% w/w of TS-530, and 68.13% w/w of ST-BAS. The cement composition has a consistency of 28mm. The enamel and dentin bond strength tests were conducted using this cement composition by following the procedures in Example 1 except the enamel surface was prepared with a fine diamond bur instead of 600 grit SiC paper. An enamel bond strength of 17.8 ± 3.2 MPa and a dentin bond strength of 23.8 ± 5.9 MPa were obtained. The aged cement composition after being subjected to accelerated aging at 42°C for 8 weeks yielded an enamel bond strength of 21.5 ± 6.2 MPa and a dentin bond strength of 21.6 ± 6.9 MPa.

### EXAMPLE 4

First, a homogeneous resin mixture (**4A**) was made of the following ingredients: 29.60% w/w of GDMA-P, 24.66% w/w of HEMA, 12.83% w/w of GDMA, 4.93% w/w of ETMPTA, 13.81% w/w of Bis-GMA, 12.83% w/w of UDMA, 0.99% w/w of ODMAB, 0.30% w/w of CQ, and 0.06% w/w of MEHQ. Then, a cement paste composition was made by blending the following ingredients together: 27% w/w of above resin mixture (**4A**), 1.87% w/w of ST-OX-50, 3.00% w/w of TS-530, and 68.13% w/w of ST-BAS. The cement composition had a consistency of 29mm. The enamel and dentin bond strength tests were conducted using this cement composition by following the procedures in Example 3. An enamel bond strength of 22.5 ± 10.6 MPa and a dentin bond strength of 24.0 ± 6.7 MPa were obtained. The aged cement composition after being subjected to accelerated aging at 42°C for 8 weeks yielded an enamel bond strength of 20.9 ± 3.2 MPa and a dentin bond strength of 19.7 ± 4.1 MPa.

### EXAMPLE 5

First, a homogeneous resin mixture(**5A**) was made of the following ingredients: 34.539% w/w of GDMA-P, 29.609% w/w of HEMA, 7.89% w/w of GDMA, 4.93% w/w of ETMPTA, 21.70% w/w of Bis-GMA, 0.99% w/w of ODMAB, 0.30% w/w of CQ, and 0.06% w/w of MEHQ. Then, a cement paste composition was made by blending the following ingredients together: 30.00% w/w of above resin mixture (**5A**), 1.81% w/w of ST-OX-50, 2.00% w/w of TS-530, and 66.19% w/w of ST-BAS. The cement composition had a consistency of 35mm. The enamel bond strength test was conducted by following the procedures in Example 3. An enamel bond strength of 24.1 ± 1.9 MPa was obtained.

### EXAMPLE 6

First, a homogeneous resin mixture(**6A**) was made of the following ingredients: 34.73% w/w of GDMA-P, 24.73% w/w of HEMA, 14.84% w/w of GDMA, 4.95% w/w of ETMPTA, 14.84% w/w of Bis-GMA, 14.84% w/w of UDMA, 0.99% w/w of ODMAB, 0.30% w/w of CQ, and 0.06% w/w of MEHQ. Then, a cement paste composition was made by blending the following ingredients together: 27.00% w/w of above resin mixture (**6A**), 1.87% w/w of ST-OX-50, 3.00% w/w of TS-530, and 68.13% w/w of ST-BAS. The enamel bond strength test was conducted by following the procedures in Example 3. An enamel bond strength of 20.6 ± 3.2 MPa was obtained. The aged cement composition after being subjected to accelerated aging at 42°C for 8 weeks yielded an enamel bond strength of 24.6 ± 6.6 MPa and a dentin bond strength of 12.5 ± 5.1 MPa.

### EXAMPLE 7

First, a homogeneous resin mixture(**7A**) was made of the following ingredients: 24.66% w/w of GDMA-P, 24.66% w/w of HEMA, 12.83% w/w of GDMA, 4.93% w/w of ETMPTA, 13.81% w/w of Bis-GMA, 13.81% w/w of UDMA, 3.95% w/w of ethanol, 0.99% w/w of ODMAB, 0.30% w/w of CQ, and 0.06% w/w of MEHQ. Then, a cement paste composition was made by blending the following ingredients together: 26.5% w/w of above resin mixture (**7A**), 1.90% w/w of ST-OX-50, 3.00% w/w of TS-530, and 68.60% w/w of ST-BAS. The cement composition had a consistency of 29mm. The enamel bond strength test was conducted by following the procedures in Example 3. An enamel bond strength of 22.3 ± 6.0 MPa was obtained.

### EXAMPLE 8

First, a homogeneous resin mixture(**8A**) was made of the following ingredients: 24.66% w/w of GDMA-P, 24.66% w/w of HEMA, 11.84% w/w of GDMA, 4.93% w/w of ETMPTA, 12.33% w/w of Bis-GMA, 12.33% w/w of UDMA, 7.89% w/w of de-ionized water, 0.99% w/w of ODMAB, 0.30% w/w of CQ, and 0.06% w/w of MEHQ. Then, a cement paste composition was made by blending the following ingredients together: 26.5% w/w of above resin mixture (**8A**), 1.90% w/w of ST-OX-50, 3.00% w/w of TS-530, and 68.60% w/w of ST-BAS. The cement composition had a consistency of 38mm. The enamel bond strength test was conducted by following the procedures in Example 3. An enamel bond strength of 23.8 ± 5.2 MPa was obtained. The aged cement composition after being subjected to accelerated aging at 42°C for 8 weeks yielded an enamel bond strength of 21.7 ± 5.1 MPa and a dentin bond strength of 15.1 ± 4.2 MPa.

### EXAMPLE 9

First, a homogeneous resin mixture(**9A**) was made of the following ingredients: 24.66% w/w of GDMA-P, 24.66% w/w of HEMA, 14.80% w/w of GDMA, 4.93% w/w of ETMPTA, 29.60% w/w of Bis-GMA, 0.99% w/w of ODMAB, 0.30% w/w of CQ, 0.06% w/w of MEHQ, and 0.99% w/w of UV-9. Then, a cement paste composition was made by blending the following ingredients together: 27.0% w/w of above resin mixture (**9A**), 1.87% w/w of ST-OX-50, 3.00% w/w of TS-530, and 68.13% w/w of ST-BAS. The cement composition had a consistency of 32mm. The enamel bond strength test was conducted by following the procedures in Example 3. An enamel bond strength of 21.7 ± 4.5 MPa was obtained.

### EXAMPLE 10

First, a homogeneous resin mixture(**10A**) was made of the following ingredients: 24.66% w/w of GDMA-P, 24.66% w/w of HEMA, 14.80% w/w of AHPMA, 4.93% w/w of ETMPTA, 6.91% w/w of Bis-GMA, 22.69% w/w of UDMA, 0.99% w/w of ODMAB, 0.30% w/w of CQ, and 0.06% w/w of MEHQ. Then, a cement paste composition was made by blending the following ingredients together: 27.0% w/w of above resin mixture (**10A**), 1.87% w/w of ST-OX-50, 3.00% w/w of TS-530, and 68.13% w/w of ST-BAS. The cement composition had a consistency of 32mm. The enamel bond strength test was conducted by following the procedures in Example 3. An enamel bond strength of 24.2 ± 8.2 MPa was obtained.

### EXAMPLE 11

First, a homogeneous resin mixture(**11A**) was made of the following ingredients: 24.42% w/w of GDMA-P, 24.42% w/w of HEMA, 14.65% w/w of GDMA, 4.88% w/w of ETMPTA, 6.84% w/w of Bis-GMA, 22.47% w/w of UDMA, 0.98% w/w of ODMAB, 0.29% w/w of CQ, 0.06% w/w of MEHQ, and 0.98% w/w of UV-9. Then, a cement paste composition was made by blending the following ingredients together: 27.0% w/w of above resin mixture (**11A**), 1.87% w/w of ST-OX-50, 3.00% w/w of TS-530, and 68.13% w/w of ST-BAS. The cement composition had a consistency of 36mm. The enamel bond strength test was conducted by following the procedures in Example 3. An enamel bond strength of 26.0 ± 3.4 MPa was obtained. When the enamel surface was further etched with Kerr Gel Etchant (37% phosphoric acid, Kerr, CA) for 15 seconds (subsequently rinsed with water and air dried with compressed air using a dental air syringe), an enamel bond strength of 35.2 ± 4.0 MPa was obtained with this cement composition.

This cement composition was also used to bond to Vitablocs® Mark II porcelain or ceramic substrate (CA, Vident) designed specifically for CEREC® CAD/CAM machine. The Vitablocs® Mark II ceramic substrate was embedded in cold cure acrylics and the bonding was conducted by following the procedures of Example 1. When the ceramic substrate was prepared by just sanding with 600 grit SiC paper without further preparation, a bond strength of 19.1 ± 4.4 MPa was obtained with the cement composition, demonstrating good self-adhesive property of the inventive composition toward the ceramic substrate. When the ceramic substrate was further etched with HF etchant (Ceram-Etch, 9.5% hydrofluoric acid, Gresco Products, TX) for 1 minute (subsequently rinsed with water and air dried with compressed air using a dental air syringe), a bond strength of 28.2 ± 5.7 MPa was obtained with the cement composition. When the ceramic substrate was further conditioned with Kerr Silane Primer (a primer for ceramic substrate, Kerr, CA) after first being etched with HF etchant, a bond strength of 31.7 ± 4.8 MPa was obtained with the cement composition, showing only marginal improvement over that obtained with just HF etching. When the ceramic substrate was first air-abraded with 50 micron aluminum oxide particles followed by etching with HF etchant, a bond strength of 28.2 ± 9.5 MPa was obtained with the cement composition, not much different from that when air-abrasion was omitted. When the ceramic substrate was first air-abraded with 50 micron aluminum oxide particles followed by etching with HF etchant and then conditioning with silane primer, a bond strength of 27.8 ± 4.6 MPa was obtained with the cement composition. Therefore, merely etching with HF etchant without any other surface treatment was sufficient to establish an excellent adhesion to porcelain or ceramic substrate with current cement composition.

### EXAMPLE 12

First, a homogeneous resin mixture (**12A**) was made of the following ingredients: 24.66% w/w of PMGDMA, 24.66% w/w of HEMA, 24.66% w/w of TEGDMA, 9.87% w/w of Bis-GMA, 14.80% w/w of UDMA, 0.99% w/w of ODMAB, 0.30% w/w of CQ, and 0.06% w/w of MEHQ. Then, a cement paste composition was made by blending the following ingredients together: 27.0% w/w of above resin mixture (**12A**), 1.87% w/w of ST-OX-50, 3.00% w/w of TS-530, and 68.13% w/w of ST-BAS. The cement composition has a consistency of 26mm. The enamel bond strength test was conducted by following the procedures in Example 3 except the enamel surface was etched with Kerr Gel Etchant for 15 seconds (subsequently rinsed with water and air dried with compressed air using a dental air syringe), and an enamel bond strength of 40.3 ± 11.7 MPa was obtained with this cement composition. This high enamel bond strength was achieved without any adhesive pretreatment on the enamel substrate.

This cement composition was also used to bond to Vitablocs® Mark II porcelain or ceramic substrate (CA, Vident) following the procedures of Example 11. When the ceramic substrate was just etched with HF etchant for 1 minute (subsequently rinsed with water and air dried with compressed air using a dental air syringe), a bond strength of 30.5 ± 5.5 MPa was obtained with the cement composition. When the ceramic substrate was further conditioned with Kerr Silane Primer after first being etched with HF etchant, a bond strength of 29.6 ± 3.7 MPa was obtained with the cement composition, showing no improvement over that obtained with just HF etching. Therefore, merely etching with HF etchant without any other surface treatment was sufficient to establish an excellent adhesion to porcelain or ceramic substrate with current cement composition.

Much simplified cementation procedures and significant time savings will result when the method and composition is used to adhere a dental prosthetic device such as a veneer, an inlay, an onlay or a crown to tooth structure. The cement composition of the current invention is self-adhering to tooth structure and the bonding surface of the prosthetic device. As a result, the steps of applying a primer/adhesive to both tooth structure and the bonding surface of the prosthetic device are omitted at the time of cementation. The cementation procedure can be further simplified by omitting the step of etching the tooth surface and/or the step of etching the bonding surface of prosthetic device.

While the present invention has been illustrated by the description of one or more embodiments thereof, and while the embodiments have been described in considerable detail, additional advantages and modifications will readily appear to those skilled in the art.

## Claims

1. A dental cementation kit comprising a light-curable single-part self-adhering cement composition, instructions for using the cement composition, and optionally one or two compositions selected from the group consisting of an acidic etchant for etching tooth structure, and an acidic etchant for etching a bonding surface of a dental prosthetic device;
wherein the light-curable single-component self-adhering cement composition comprises:
(i) at least one acidic polymerizable monomer having at least one acidic moiety and at least one ethylenically unsaturated group,
(ii) at least one non-acidic polymerizable monomer having at least one ethylenically unsaturated group,
(iii) at least one photo-initiator, and
(iv) at least one filler, wherein the weight ratio of component (i) to component (ii) ranges from about 0.5:99.5 to about 70:30; and
wherein the instructions for using the cement composition to attach a prosthetic device to tooth structure omit the steps of applying a primer and/or adhesive to the tooth structure and the bonding surface of the prosthetic device.

2. The dental cementation kit of claim 1 with the proviso that the kit does not include a primer or an adhesive.

3. The dental cementation kit of either claim 1 or claim 2 wherein the weight ratio of component (i) to component (ii) in the cement composition ranges from about 5:95 to about 60:40, preferably from about 10:90 to about 50:50.

4. The dental cementation kit of any preceding claim wherein the concentration of component (iv) in the cement composition is in the range from about 15-95% w/w, preferably from about 50-80% w/w.

5. The dental cementation kit of any preceding claim wherein the cement composition further comprises one or more components selected from the group consisting of a colorant, a stabilizer, a UV absorber, a solvent, a fluoride-releasing compound, an antimicrobial additive, and a surfactant, and combinations thereof.

6. The dental cementation kit of any preceding claim wherein the ethylenically unsaturated group is selected from the group consisting of an acrylate, a methacrylate, acrylamide, methacrylamide, and a vinyl group.

7. The dental cementation kit of any preceding claim wherein the acidic moiety is selected from the group consisting of phosphorous-containing acidic moiety, carbon-containing acidic moiety, sulfur-containing acidic moiety, and boron-containing acidic moiety.

8. The dental cementation kit of any one of claims 1 to 6 wherein the acidic polymerizable monomer has at least one acidic moiety selected from the group consisting of 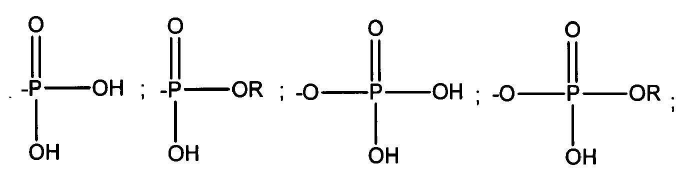 where R is an alkyl group, aryl group, or alkali metal ion.

9. The dental cementation kit of any one of claims 1 to 6 wherein the acidic polymerizable monomer is selected from the group consisting of phenyl methacryloxyethyl phosphate, glyceryldimethacrylate phosphate, dipentaerithritol pentaacrylate phosphate, methacryloyloxydecyl phosphate, pentaerithritol triacrylate phosphate, hydroxyethylmethacrylate phosphate, and bis(hydroxyethylmethacrylate) phosphate, and combinations thereof.

10. The dental cementation kit of any one of claims 1 to 6 wherein the acidic polymerizable monomer is selected from the group consisting of 4-methacryloxyethyltrimellitic anhydride, and 4-methacryloxyethyltrimellitic acid, and a combination thereof.

11. The dental cementation kit of any one of claims 1 to 6 wherein the acidic polymerizable monomer is an addition product of a mono- or di-anhydride compound with a hydroxyalkylmethacrylate compound.

12. The dental cementation kit of any preceding claim wherein the at least one filler is selected from the group consisting of inorganic metal, salt, oxide, nitride, silicate glass, aluminosilicate glass, aluminoborosilicate glass, fluoroaluminosilicate glass, quartz, fumed silica, colloidal silica, precipitated silica, zirconia-silica, polymeric filler, and polymerized composite filler with inorganic particles, and combinations thereof.

13. The dental cementation kit of claim 12 wherein the metals, salts, oxides, silicate glass, aluminosilicate glass, aluminoborosilicate glass, and fluoroaluminosilicate glass contains an element selected from the group consisting of Sr, Y, Zr, Ba, La, Hf, Zn, Bi, W, and rare earth metal, and combinations thereof.

14. The dental cementation kit of any preceding claim wherein the acidic etchant for etching the tooth structure comprises an acid selected from the group consisting of phosphoric acid, citric acid, maleic acid, phosphonic acid, and nitric acid,preferab ly phosphoric acid.

15. The dental cementation kit of any preceding claim wherein the acidic etchant for etching the bonding surface of the prosthetic device comprises an acid selected from the group consisting of hydrofluoric acid, phosphoric acid, citric acid, maleic acid, phosphonic acid, and nitric acid, preferably hydrofluoric acid.

16. The dental cementation kit of any one of claims 1 to 13 wherein the acidic etchant for etching tooth structure and/or the acidic etchant for etching the bonding surface of a prosthetic device is omitted from the kit and wherein the instructions for using the cement composition further omits the corresponding etching step for the acidic etchant omitted from the kit.
